# EUROPEAN PATENT SPECIFICATION

(11) **EP 1 365 019 B1**
(45) Date of publication and mention of the grant of the patent: **26.07.2006**
(21) Application number: 02100543.4
(22) Date of filing: 21.05.2002
(51) Int. Cl.: C12N 9/58, C12P 21/00, C12N 1/14, C12R 1/645, C12R 1/785

(54) **Fermentative production of microbial milk clotting protease**
Fermentative Herstellung von mikrobieller milchgerinnender Protease
Production par fermentation de protéase microbienne pour le caillage du lait

(43) Date of publication of application: 26.11.2003
(73) Proprietor: DSM IP Assets B.V., 6411 TE Heerlen (NL)
(72) Inventor: Hensing, Marcus Clemens Marie, 59700 Marq-en-Baroeul (FR)
(74) Representative: Matulewicz, Emil Rudolf Antonius

(56) References cited:
- US-A- 3 607 655
- US-A- 5 914 259
- SEKER SULE ET AL: "Production of microbial rennin from Mucor miehei in a continuously fed fermenter." ENZYME AND MICROBIAL TECHNOLOGY, vol. 23, no. 7-8, 15 November 1998 (1998-11-15), pages 469-474, XP008009545 ISSN: 0141-0229
- AYHAN F ET AL: "THE EFFECT OF FERMENTATION PARAMETERS ON THE PRODUCTION OF MUCOR MIEHEI ACID PROTEASE IN A CHEMICALLY DEFINED MEDIUM" JOURNAL OF CHEMICAL TECHNOLOGY AND BIOTECHNOLOGY. (INTERNATIONAL JOURNAL OF BIOTECHNICAL AND CHEMICAL PROCESSES), ELSEVIER APPLIED SCIENCE PUBLISHERS. BARKING, GB, vol. 76, no. 2, 1 February 2001 (2001-02-01), pages 153-160, XP001001967 ISSN: 0268-2575
- THAKUR M S ET AL: "PRODUCTION OF FUNGAL RENNET BY MUCOR-MIEHEI USING SOLID STATE FERMENTATION" APPLIED MICROBIOLOGY AND BIOTECHNOLOGY, vol. 32, no. 4, 1990, pages 409-413, XP008009547 ISSN: 0175-7598
- SOMKUTI G A ET AL: "Acid protease synthesis by Mucor pusillus in chemically defined media." JOURNAL OF BACTERIOLOGY. UNITED STATES APR 1968, vol. 95, no. 4, April 1968 (1968-04), pages 1415-1418, XP008009709 ISSN: 0021-9193
- GHAREIB M ET AL: "Production of intracellular milk-clotting enzyme in submerged cultures of Fusarium subglutinans." ACTA MICROBIOLOGICA POLONICA, vol. 50, no. 2, 2001, pages 139-147, XP008009540 ISSN: 0137-1320

## Description

The present invention relates to a process for the fermentative production of milk clotting protease by fungi and the use of said protease in cheese making.

Conventional methods for making cheese involved the use of calf rennet for coagulating milk. Due to a worldwide shortage of calf rennet in recent decades, alternative sources to supply milk-clotting enzymes have been investigated. Nowadays, microbial rennets are used as substitute for calf rennets. Three important fungal sources widely used are *Rhizomucor miehei, Rhizomucor pusillus* and *Endothia parasitica* (renamed *Cryphonectria parasitica)*. The rennet of *M. miehe*i has generally been preferred as a substitute for true calf rennet because of its specific properties similar to calf rennet [Escobar et al Enzyme Microb. Technol. 1993 vol. 15 1009-1013]. *M. miehei* is a thermophilic zygomycete and produces an acid protease with a molecular weight of 34,000-39,000. This acid protease reveals relatively high milk coagulating activity and relatively low a-specific proteolytic activity.

Recently, a fermentation process has been disclosed that describes the production of microbial rennin from *Mucor miehei* in a continuously fed fermentor. In this document the effects of medium pH, mixing and dilution rates, and feed of carbon source on milk-clotting activity were elaborated, and optimal parameters were determined for small scale fermentation process wherein glucose is continuously fed [S. Seker et al. 1999, J. Chem. Technol. Biotechnol. 74: 527-532]. However, although the acid protease from *Mucor miehei* has already been used for more than 20 years in industrial cheese making, there are still a number of problems associated with industrial production (fermentation) process.

In literature, the effects of different nitrogen sources on the production have intensively been studied on small scale (up to 5 l). From several studies it was concluded that *M. miehei* does need a complex nitrogen source, and that this nitrogen source in combination with casein, possitively affects the production of the milk clotting protease, since casein has been described to induce protein production. Furthermore, it was shown that among different nitrogen sources, ammonium nitrate supported only moderate enzyme formation [S. Thakur et al 1990]. On the other hand, peptone as nitrogen source had a positive effect on milk clotting enzyme production. In general, organic nitrogen sources are preferred over inorganic nitrogen sources such as ammonia [Mashaly et al 1983. Egyptian J. Dairy Sci. 11:255-265]. Also it has been shown that the presence of ammonia in the medium repressed enzyme activity in the absence of casein [Lasure, L.L., *et al*. 1980, Mycologia 72: 83-86]. These published studies suggest the use of a nitrogen source other than ammonia when developing an optimal fermentation process for the production of milk-clotting enzyme at small production scale.

Seker *et al.* (Enzyme and Microbioal Technology 23; 469-474, 1998) and Ayhan *et al.* (J.Chem.Technol.Biotechnol. 76:153-160, 2001) describe the production of the microbial rennin from *Mucor miehei,* respectively in continous and batch processes, using a medium containing ammonium and a complex nitrogen source (yeast extract). The effect of varying the ammonium concentration was not investigated in these publications.

The main objective of present invention is to provide an optimised fermentation process for the industrial production of milk-clotting protease with high enzyme yield. For industrial production fermentors of more than 1 m³ , preferably of more than 2.5 m³ are used. In general the fermentors are smaller than 250 m³.

Surprisingly it has been found that using a (extended) fed batch fermentation process wherein the concentration of ammonia in the fermentation broth is maintained at 0.2-2 g/L during the production of the milk clotting protease, the yield of said protease is increased when compared to prior art processes wherein ammonia is not maintained at said level. The present invention therefore relates to a novel process for the production of microbial milk clotting protease and the use of this protease for example in cheese making.

In accordance with the present invention a microbial milk clotting protease is prepared by the fermentation process that comprises the following steps. Pre-fermenting an inoculum of a fungus on an assimilable carbon source in suitable nutrient medium. Introducing said inoculum into a fermentor, and fermenting said fungus in a nutrient medium comprising an assimilable carbon source, an assimilable nitrogen source and essential minerals using a fed batch method.

The process comprises the use of a micro-organism that is able to produce milk clotting protease. This micro-organism can be a fungus, more preferably a strain selected from the genus *Rhizomucor* or a strain from *Cryphonectria parasitica.*

The fermentation comprises a prefermentation of an inoculum of above mentioned strain, followed by fermentation resulting in production of milk-clotting protease at high yield. During inoculum fermentation, an inoculum of said fungus can be cultured on a suitable nutrient medium, indicated as inoculation medium, containing an assimilable carbon source, an assimilable nitrogen source, an assimilable phosphate source, and minerals. In order to achieve a large volume of the inoculum sufficient to start the fed batch fermentation in the main fermentor at high/industrial scale (more than 1 m³.), several inoculum propagation steps are required, each carried out in a volume greater than the previous step, and subsequently the inoculum is introduced into a fermentor wherein the main fermentation is carried out.

The nutrient medium in the main fermentor (batch medium) comprises an assimilable carbon source, assimilable nitrogen source and essential minerals. The carbon source can be the same or different from the one used in the inoculum medium. Assimilable carbon sources can be mono-, di- or polysaccharides such as glucose, sucrose, lactose, or whey, (corn) starch, wheat bran, starch malt extract or hydrolysed forms hereof or combinations hereof. Preferably, glucose can be used. The concentration present in the batch medium at the start of the fermentation can be more than 5 g/l. Preferably 50-100g/l is present. Feeding of assimilable carbon source into the fermentor is in such a way that the concentration in the broth is maintained preferably less than 5 g/l during production. More preferably 1-2 g/l. In a most preferred embodiment of present invention, depending on the micro-organism used, the metabolisable carbon source can be maintained at limiting concentration (f.e. glucose concentration is between 0-1 g/l) in order to obtain best yields.

Suitable nitrogen sources can comprise yeast hydrolysates, primary yeast, soybean meal, cotton seed flour, casein or hydrolysate hereof, corn steep liquor/solids, peptone, meat extract, rice bran, soy flour and/or soy protein. Alternatively, inorganic nitrogen sources such as ammonia or salts thereof, or organic nitrogen sources, such as urea and/or amino acids, can be used. In a preferred embodiment of the invention, the nitrogen source can be a combination of any of the above mentioned nitrogen sources with ammonia (added for example as ammonium-hydroxide or -sulphate) that is maintained at a concentration of 0.2-2 g/l in the fermentation broth during production of the milk clotting protease. Preferably the ammonia concentration is between 0.5-1.5 g/l. In the most preferred embodiment, ammonia can be used in combination with soy flour, wherein the concentration soyflour is between 2-10 g/L

Apart from the assimilable carbon and nitrogen sources, the batch medium can be provided with a variety of organic or inorganic compounds which provide sulfur, phosphorus, iron, magnesium, and other elements essential for cell growth, viability and production of milk clotting protease. Typical nutrient medium formulations are illustrated in the Examples.

During fermentation, the dissolved oxygen tension is kept at such a rate that oxygen is not limiting. Aeration of the culture medium during fermentation can be achieved by bubbling air through the culture. The overpressure is preferably between 0.5-2.0 bar, more preferably 1.2-1.4 bar. Alternatively or in addition to aeration, the broth can be agitated. This can be achieved in various ways, although agitation by stirring is often preferred. Preferably agitation and/or aeration should keep the dissolved oxygen tension above 20% air saturation level. The temperature of the fermentation process can vary depending on the fungus used. Preferably, a temperature between 25-47°C can be used. More preferably the temperature can be 35-42°C.

The pH should preferably be kept constant throughout the fermentation process. This can be achieved by using a buffered culture medium, or by using a balance of acid and alkali. A feed of phosphoric acid or NH4OH solution can be applied. The pH is usually between 3.0-8.0 preferably the pH is kept between 4.5-5.5.

According to the invention the main fermentation is carried out using a fed batch method, preferably an extended fed batch can be used. For the purpose of present invention the term fed batch implies fermentation in which one or more of the nutrients are introduced into the culture medium not only initially but also during the fermentation and the broth is extracted from the fermentation tank only at the end of the fermentation process. The term extended fed batch implies a fermentation process similar to a fed batch with the difference that the broth is not withdrawn from the fermentation tank only at the end of the fermentation but part of the broth is periodically withdrawn from the fermentor, preferably with a volume of 5-60% of the broth volume. Feeding, in order to maintain the concentration at a preferred value can occur once or several times with intervals, or continuously at a constant or decreasing or increasing rate. Preferably, feeding can be increased during the first period of time, whereas it is kept constant until the end of the fermentation. It may be clear to a person skilled in the art that the feeding of nutrient(s) into the fermentor can be continuously adjusted dependent on the monitored concentration of said nutrient in the fermentation broth, in order to maintain the desired concentration.

After fermentation, said milk clotting protease is recovered from the fermentation broth, and purified according to methods known in the art. Protease activity can be measured according to the International Dairy federation procedure.

The microbial milk clotting protease as prepared according to the process of present invention can be used in the making of ripened cheeses such as Cheddar type cheeses, Swiss cheese and other co-called hard and soft cheeses, and for any other types of cheeses wherein a milk-coagulating preparation is used.

### Example 1

### Fermentative production of microbial milk clotting protease

For Inoculum preparation, one millilitre of a deep-frozen culture of *Rhizomucor miehei* NRRL-A13042 was diluted in 9 millilitre of a physiological salt solution (0.8% NaCI in demineralised water). 1 ml of this suspension was plated on a PDA agar slant prepared in a shake flask (PDA composition 39 g/l of potato dextrose agar).

| components | Fermentation 1 (comparative) | | Fermentation 2 | |
|---|---|---|---|---|
| | Batch Medium | Feed medium | Batch medium | Feed medium |
| Glucose | 50 | 100 | 50 | 100 |
| Soy flour | 50 | 65 | 50 | 20 |
| Hydrolysed starch | 40 | - | - | - |
| NH₄OH | - | - | - | - |
| (NH₄)₂SO₄ | 2 | - | 2 | - |
| KH₂PO₄ | 4 | 5 | 4 | 5 |
| CaCl₂.2H₂O | 0.1 | 0.1 | 0.1 | 0.1 |
| Citric acid | 1 | 1 | 1 | 1 |
| MgSO₄.7H₂O | 1 | 1 | 1 | 1 |
| FeSO₄.7H₂O | 0.1 | 0.1 | 0.1 | 0.1 |
| MnSO₄.1H₂O | 0.01 | 0.01 | 0.01 | 0.01 |
| Zn SO₄.7H₂O | 0.01 | 0.01 | 0.01 | 0.01 |
| Antifoam | 0.2 | 0.2 | 0.2 | 0.2 |

The slants were incubated for 4 days at 39 °C. The formed spores were harvested by adding 100 ml of water containing 1 g/l of Tween80 and glass beads, followed by gentle shaking of the flask.20 ml of the spore suspension was added to a 1 litre shake flask containing 200 ml of the following inoculation medium (glucose. 1H₂O 20 g/l, yeast extract 5 g/l, Soy flour 15 g/l, K₂HPO₄ 1 g/l with a pH of 6.2). The shake flask was incubated for 30 hours in a rotary shaker at 39°C at 250 rpm. Several rounds of upscaling were performed in order to inoculate the main fermentor (3000 I).

The main fermentation for the production of milk clotting protease is performed in a extended fed-batch fermentation in a 3000 litre pilot plant fermentor. Batch medium for main fermentation and the feed medium contained the following components (g/kg) for subsequently the comparative fermentation process (Fermentation 1) and the fermentation process of present invention (Fermentation 2). Next to the feed in Fermentation 2, a separate feed of pure ammonium hydroxide (14N) is fed into the fermentor at a rate so that the concentration ammonia in the broth is maintained at 1.5 g/l.

The feed (both feed medium and ammonium hydroxide feed) were launched when the glucose concentration in the broth was below 5g/l. The feed rate was controlled in a way that the glucose concentration was maintained at 1 g/l. In a comparative example no separate ammonium hydroxide feed was launched whereas in the example showing present invention a separate ammonium hydroxide feed was controlled in a way the ammonia concentration was maintained at approximately 1.5 g/l. Batchwise withdrawals were taken after 48 hours every 12 hours so that the weight of the fermentor was brought at 1800 kg. The main fermentation was carried out over a period of 140 hours. Samples for detecting milk clotting enzyme were taken approximately every 20 hours until the end of the fermentation.

The pH was controlled at 5.25+/-0.25. The temperature was kept at 39°C. Aeration and agitation were set to maintain an aerobic fermentation. The milk clotting activity was measured according to the International Dairy federation procedure. The relative yield of milk clotting protease in the comparative fermentation was set at 100%. An improvement in yield was observed when applying the fermentation process of current invention, since it was determined that the relative yield varied from 2.25 times the yield as measured in the comparative fermentation over the first 40 hours till 1.50 - 1.75 times over the period from 60-140 hours.

## Claims

1. A process for producing a milk clotting protease, which comprises:
a) fermenting a fungus in a nutrient medium comprising an assimilable nitrogen source using a fed batch method, and
b) maintaining the ammonium concentration at 0.2-2 g/l, and
c) recovering said milk clotting protease.

2. The process according to claim 1 wherein said fungus is a strain selected from the group consisting of *Rhizomucor miehei, Rhizomucor pusillus, Cryphonectria parasitica.*

3. The process according to claim 1 wherein the fed batch is a extended fed batch with batchwise withdrawals of part of the fermentation broth.

4. The process according to any of claims 1 to 3, which is carried out in a fermentor of more than 1 m³.

5. The process according to any of claims 1 to 4, wherein ammonium hydroxide is fed to maintain the ammonium concentration.

## Patentansprüche

1. Verfahren zur Herstellung einer milchgerinnenden Protease, das folgendes umfasst:
a) Fermentieren eines Pilzes nach dem Zulaufverfahren ("Fed-Batch") in einem Nährmedium, das eine assimilierbare Stickstoffquelle umfasst, und
b) Aufrechterhalten einer Ammoniumkonzentration von 0,2-2g/l und
c) Gewinnen der milchgerinnenden Protease.

2. Verfahren nach Anspruch 1, wobei der Pilz ein Stamm ist, der gewählt wurde aus der Gruppe, die aus Rhizomucor miehei, Rhizomucor pusillus, Cryphonectria parasitica besteht.

3. Verfahren nach Anspruch 1, wobei das Zulaufverfahren (Fed Batch) ein erweitertes Zulaufverfahren ("extended fed batch") ist, bei dem satzweise Fermentationsbrühe abgezogen wird.

4. Verfahren nach einem der Ansprüche 1 bis 3, das in einem Fermenter von mehr als 1 m³ durchgeführt wird.

5. Verfahren nach einem der Ansprüche 1 bis 4, wobei Ammoniumhydroxid eingespeist wird, um die Ammoniumkonzentration aufrechtzuerhalten.

## Revendications

1. Procédé de production d'une protéase pour le caillage du lait, qui comprend:
a) la fermentation d'un champignon dans un milieu nutritif comprenant une source d'azote assimilable utilisant un procédé discontinu à alimentation, et
b) le maintien de la concentration d'ammonium à 0,2-2 g/l, et
c) la récupération de ladite protéase pour le caillage du lait.

2. Procédé selon la revendication 1, dans lequel ledit champignon est une souche sélectionnée parmi le groupe composé de *Rhizomucor miehei, Rhizomucor pusillus, Cryphonectria parasitica.*

3. Procédé selon la revendication 1, dans lequel le lot alimenté est un lot alimenté étendu avec retraits séquentiels d'une partie du bouillon de fermentation.

4. Procédé selon l'une quelconque des revendications 1 à 3, qui est mis en oeuvre dans un fermenteur de plus de 1 m³.

5. Procédé selon l'une quelconque des revendications 1 à 4, dans lequel de l'hydroxyde d'ammonium est apporté pour maintenir la concentration d'ammonium.
